# EUROPÄISCHE PATENTSCHRIFT

(11) **EP 0 622 376 B1**
(45) Veröffentlichungstag und Bekanntmachung des Hinweises auf die Patenterteilung: **08.08.2001**
(21) Anmeldenummer: 94106196.2
(22) Anmeldetag: 21.04.1994
(51) Int. Cl.: C07K 14/62, A61K 38/28

(54) **Amorphe monosphärische Formen von Insulinderivaten**
Amorphous monospheric forms of insulin derivatives
Amorphes formes monosphériques de dérivés d'insuline

(30) Priorität: 27.04.1993 DE 4313702
(43) Veröffentlichungstag der Anmeldung: 02.11.1994
(73) Patentinhaber: HOECHST AKTIENGESELLSCHAFT, 65929 Frankfurt am Main (DE)
(72) Erfinder: Obermeier, Rainer, Dr., D-65795 Hattersheim (DE); Sabel, Walter, D-65520 Bad Camberg (DE); Deil, Peter, D-65931 Frankfurt am Main (DE); Geisen, Karl, Dr., D-60318 Frankfurt am Main (DE)

(56) Entgegenhaltungen:
- CHEMICAL ABSTRACTS, Band 118, Nr. 11, 15. M rz 1993, Columbus, Ohio, USA HU,Y. et al."Crystallographic study on highly stable insulins-crystallization and preliminary cristallographic analysis of A21-Ser mutant" Seite 97, Spalte 2, Nr. 94 501u; & Chin.Sci.Bull. 1992, 37(16), 1390-3 (Eng).
- CHEMICAL ABSTRACTS, Band 114, Nr. 25, 24. Juni 1991, Columbus, Ohio, USA MARKUSSEN,J.et al."Prolonged- acting insulins, stable in acid solution. Crystallizability and biological potency of insulins substituted in positions A21, B27 and B30." Seite 140, Spalten 1,2; Nr. 241 014k; & Pept.,Proc.Eur.Pept.Symp. 20th, 1988 (Pub.1989), 658-60 (Eng).
- CHEMICAL ABSTRACTS, Band 97, Nr. 10, 6. September 1982, Columbus, Ohio, USA BIALOBRZESKA,A. et al."Effect of crystallization parameters on the quality of insulin." Seiten 414,415, Spalten 2(1), Nr. 78 828y; & Acta Pol.Pharm. 1981, 38(6), 685-93 (Pol.).

## Beschreibung

Insulin ist ein Polypeptid, das aus 51 Aminosäureresten aufgebaut ist. Die sogenannte A (acidic)-Kette besteht aus 21, die B (basic)-Kette aus 30 Aminosäureresten. Die beiden Ketten werden im nativen Molekül durch 2 Cystinbrücken verknüpft. In der A-Kette findet sich eine weitere Disulfidbrücke zwischen den Cysteinresten in Position 6 und 11.

Seit den Arbeiten von D.A. Scott (Biochem. J.,(1934), 28, 1592) sind Verfahren zur Kristallisation von Insulinen bekannt. Durch die Zugabe von Zn²⁺-lonen zu den üblichen Kristallisationspuffern bildet Insulin bei seinem isoelektrischen Punkt von pH 5,4 bevorzugt eine rhomboedrische Kristallform aus. Die Elementarzelle enthält ein hexameres Insulin, das 2 Zn²⁺-lonen komplexiert.

Insulin vom Menschen, Schwein oder Rind ist im präparativen Maßstab leicht kristallisierbar, während sich manche Insulinderivate wie zum Beispiel Di-Arg-(B31-32)-Humaninsulin nur in Gegenwart von Phenol kristallisieren läßt.

Bei der Herstellung und Reinigung von Insulinderivaten ist es oft notwendig, das gereinigte Material ohne Phenolzusatz zu isolieren, damit die Herstellung galenischer Zubereitungen nicht beeinträchtigt. Für präparative Zwecke bietet sich für dieses Insulinderivat eine isoelektrische Fällung mit anschließender Zentrifugation und Gefriertrocknung an. Dabei entsteht ein flockiges Insulinlyophilisat, das schwer zu handhaben ist und wie alle gefriergetrockneten Proteine auf Grund seines hygroskopischen Verhaltens nur schwierig zu dosieren ist.

Aufgabe der vorliegenden Erfindung war es, ein Verfahren zu entwickeln, mit dem sich schlecht kristallisierbare Insulinderivate in fester Form abtrennen lassen.

Es wurde nun ein Verfahren gefunden, durch das sich Insulinderivate aus Lösungen isolieren lassen, das dadurch gekennzeichnet ist, daß man
A) das Insulinderivat in einem n-Propanol-Puffer-Gemisch löst, wobei der n-Propanol-Gehalt bezogen auf Wasser mehr als 15 % und der pH-Wert 4,5 bis 6,5 beträgt, und
B) die erhaltene Lösung mit Wasser verdünnt, so daß der n-Propanol-Gehalt bezogen auf Wasser kleiner als 15 % ist, wobei eine gemäß Debye-Scherrer-Röntgenanalyse amorphe monosphärische Form des Insulinderivats aus der Lösung ausfällt.

Die so hergestellten Insulinderivate zeigen überraschenderweise eine amorphe monosphärische Form mit einem durchschnittlichen Durchmesser von etwa 5 *µ*m. Diese so hergestellten Insulinderivate lassen sich durch Filtration oder Zentrifugation aus Suspension gewinnen, sind im getrockneten Zustand stabil und lassen sich in entsprechenden Zubereitungen auch als stabile Suspension verwenden.

Die Erfindung betrifft ferner eine amorphe monosphärische Form der Insulinderivate, dadurch erhältlich, daß man
A) das Insulinderivat in einem n-Propanol-Puffer-Gemisch löst, wobei der n-Propanol-Gehalt bezogen auf Wasser mehr als 15 % und der pH-Wert 4,5 bis 6,5 beträgt, und
B) die erhaltene Lösung mit Wasser verdünnt, so daß der n-Propanol-Gehalt bezogen auf Wasser klein2er als 15 % ist, wobei eine gemäß Debye-Scherrer-Röntgenanalyse amorphe monosphärische Form des Insulinderivats aus der Lösung ausfällt.

Bevorzugt sind Insulinderivate der Formel I 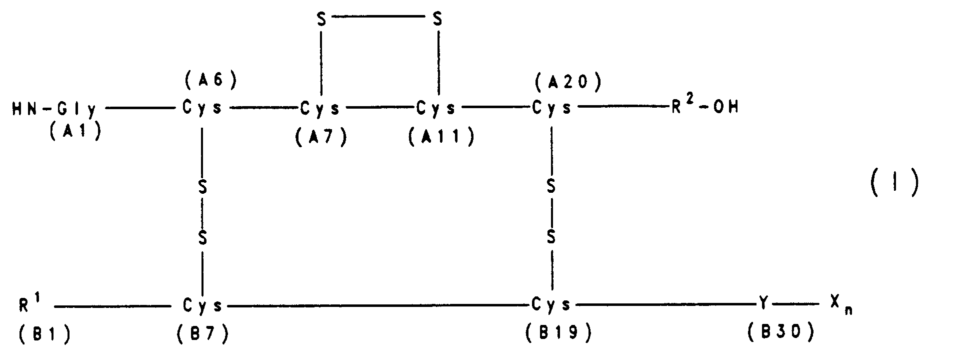 dabei ist
- X: ein genetisch kodierbarer Aminosäurerest,
- n: eine ganze Zahl von 1 bis 10,
- Y: ein genetisch kodierbarer Aminosäurerest,
- R¹: ein Phenylalaninrest oder Wasserstoffatom,
- R²: ein genetisch kodierbarer Aminosäurerest und
die Reste A2 - A20 entsprechen der Aminosäuresequenz der A-Kette von Humaninsulin, tierischem Insulin oder einem Insulinderivat, und die Reste B2 - B 29 entsprechen der Aminosäuresequenz der B-Kette von Humaninsulin, tierischem Insulin oder einem Insulinderivat.

Bevorzugt sind Insulinderivate der Formel I; dabei ist
- X: ein Aminosäurerest von Arg oder Lys,
- n: eine ganze Zahl von 1 oder 2,
- Y: ein Aminosäurerest von Ala, Thr oder Ser,
- R¹: der Aminosäurerest Phe,
- R²: ein Aminosäurerest von Asn, Ser oder Gly,
und die Reste A2 - A20 und B2 - B29 entsprechen der Aminosäuresequenz der A- und B-Ketten von Humaninsulin.

Besonders bevorzugt sind Insulinderivate der Formel I; dabei ist
- X: ein Aminosäurerest von Arg oder Lys,
- n: eine ganze Zahl von 1 oder 2,
- Y: der Aminosäurerest Thr,
- R¹: der Aminosäurerest Phe,
- R²: der Aminosäurerest von Asn oder Gly,
und die Reste A2 - A20 und B2 - B29 entsprechen der Aminosäuresequenz der A- und B-Ketten von Humaninsulin.

Die Aminosäuresequenz von Peptiden und Proteinen wird vom N-terminalen Ende der Aminosäurekette an bezeichnet. Die in Formel I in Klammern gemachten Angaben, z.B. A6, A20, B1, B7, B19 oder B30 entsprechen der Position von Aminosäureresten in den A- oder B-Ketten des Insulins.

Für den Begriff "genetisch kodierbarer Aminosäurerest" stehen die Reste der Aminosäuren Gly, Ala, Ser, Thr, Val, Leu, Ile, Asp, Asn, Glu, Gln, Cys, Met, Arg, Lys, His, Tyr, Phe, Trp, Pro und Selenocystein.

Die Begriffe "Reste A2 - A20" und "Reste B2 - B 29" von tierischem Insulin bedeuten beispielsweise die Aminosäuresequenzen von Insulin aus Rindern, Schweinen oder Hühnern verstanden.
Der Begriff Reste A2 - A20 und B2 - B29 von Insulinderivaten steht für die entsprechenden Aminosäuresequenzen von Humaninsulin, die durch den Austausch von Aminosäuren durch andere genetisch kodierbare Aminosäuren gebildet werden.

Die A-Kette von Humaninsulin hat folgende Sequenz (Seq Id No. 1):

Gly, Ile, Val, Glu, Gln, Cys, Cys, Thr, Ser, Ile, Cys, Ser, Leu,
Tyr, Gln, Leu, Glu, Asn, Tyr, Cys, Asn

Die B-Kette von Humaninsulin hat folgende Sequenz (Seq Id No. 2 ):

Phe, Val, Asn, Gln, His, Leu, Cys, Gly, Ser, His, Leu, Val, Glu,
Ala, Leu, Tyr, Leu, Val, Cys, Gly, Glu, Arg, Gly, Phe, Phe, Tyr,
Thr, Pro, Lys, Thr

Das Insulinderivat der Formel I kann mit Hilfe einer Vielzahl gentechnischer Konstrukte in Mikroorganismen gebildet werden (EP 0 489 780, EP 0 347 781, EP 0 368 187, EP 0 453 969). Die gentechnischen Konstrukte werden in Mikroorganismen wie Escherichia coli oder Streptomyceten während der Fermentation exprimiert. Die gebildeten Proteine werden im Inneren der Mikroorganismen abgelagert (EP 0 489 780) oder in die Fermentationslösung ausgeschieden.

Für das erfindungsgemäße Verfahren können Insulinderivate der Formel I in vorgereinigter Form beispielsweise nach einer Fällung oder einer chromatographischen Reinigung eingesetzt werden.

Beim Verfahrensschritt A) geht man wie folgt vor:

Die Insulinderivate werden in einem n-Propanol-Puffer-Gemisch bis zu einer Konzentration von 1 % gelöst.

Der n-Propanol-Gehalt, bezogen auf Wasser, beträgt mehr als 15 %, bevorzugt 25 bis 70 %, insbesondere 40 bis 60 %.

Der pH-Wert des n-Propanol-Puffer-Gemisches wird mit dem Puffer konstant gehalten. Geeignete Puffersubstanzen sind beispielsweise Glycin-Essigsäure-Ammoniumsulfat-Puffer. Es können im Puffer aber noch weitere Substanzen anwesend sein, wie sie für Chromatographierverfahren zur Reinigung der Insulinderivate nötig sind, wie Glycin, Glutamin oder Betain. Die Konzentration der Pufferkomponenten kann in weiten Grenzen schwanken, bevorzugt sind Konzentrationen von 0,01 bis 0,5 M.

Ferner können gegebenenfalls Zn²⁺ lonen anwesend sein. Es können beispielsweise 0,1 bis 1,0 % ZnCl₂, bevorzugt von 0,3 bis 0,5 %, zugegeben werden.

Bei Verfahrensschritt B) wird die erhaltene Lösung aus A) mit Wasser oder Puffer verdünnt. Der n-Propanol-Gehalt sollte nach der Verdünnung weniger als 15 % betragen, bevorzugt 5 bis 12 %. Die verdünnte Lösung wird langsam bei Raumtemperatur für 1 bis 5 Stunden, vorzugsweise 2 bis 3 Stunden, gerührt, wobei die amorphe monosphärische Form der Insulinderivate ausfällt. Anschließend kann die Suspension 12 bis 16 Stunden bei 4°C gelagert werden.

Dabei sedimentiert das monosphärische Insulinderivat. Nach Überprüfung des klaren Überstandes auf Abwesenheit des Insulinderivats wird dekantiert und die konzentrierte Suspension durch Zentrifugation getrennt, mit wenig Wasser gewaschen und im Vakuum bei 0°C getrocknet oder lyophilisiert.

Die n-Propanol-haltige Pufferlösung des Insulinderivates wird hergestellt, indem man das auf beliebige Art gewonnene feste Produkt im obigen Puffer bis zu einer Konzentration von 0,5 bis 1,7 %, bevorzugt 1 %, löst.

Zur Isolierung und chromatographischen Reinigung der Insulinderivate ist die Verwendung entsprechend geeigneter Puffer, die Propanol enthalten, bevorzugt, weil damit eine sofortige Ausfällung der erfindungsgemäßen Form der Insulinderivate möglich ist.

Die erfindungsgemäßen Insulinderivate sowie die entsprechenden pharmazeutischen Zubereitungen, welche diese Insulinderivate enthalten, eignen sich zur Behandlung von Diabetes mellitus.

In den folgenden Beispielen wird das erfindungsgemäße Verfahren im einzelnen beschrieben. Prozentangaben beziehen sich auf das Gewicht, sofern nicht anders angegeben.

### Beispiel 1

### Herstellung von amorphem, monosphärischen Gly-A21-di-Arg-(B31-32)-Humaninsulin

Durch Fermentation von genetisch modifizierten Escherichia-coli-Zellen (EP 0 368 187) wird Insulinderivat 1 mit folgender Aminosäuresequenz hergestellt:
Insulin 1 (Seq Id No. 3):

Phe Val Asn Gln His Leu Cys Gly Ser His Leu Val Glu Ala Leu Tyr Leu Val
Cys Gly Glu Arg Gly Phe Phe Tyr Thr Pro Lys Thr Arg Arg Gly Ile Val Glu
Gln Cys Cys Thr Ser Ile Cys Ser Leu Tyr Gln Leu Glu Asn Tyr Cys Gly

Insulin 1 entspricht der Formel I, dabei ist
- X: Arg-Arg
- n: die Zahl 2,
- Y: Thr (B30),
- R¹: Phe (B1),
- R²: Gly (A21) und

A2 - A20 die Aminosäuresequenz der A-Kette von Humaninsulin (Aminosäurereste 2 bis 20) und B2 - B29 die Aminosäuresequenz der B-Kette von Humaninsulin (Aminosäurereste 2 bis 29).

50 g ungereinigtes Insulin 1 wird in 3,3 l einer Lösung von 0,1 M Glycin, 0,05 M (NH₄)₂SO₄, 0,025 M Essigsäure und 50 % n-Propanol bei pH 5,5 gelöst und mit 3,3 l Puffer A (0,2 M Ammoniumsulfat, 0,1 M Glycin, 0,025 M Essigsäure, 5 % n-Propanol in Wasser, pH 5,5) verdünnt. Die erhaltene Mischung wird mit Wasser auf einen Propanolgehalt von weniger als 10 % verdünnt und 0,4 % ZnCl₂, bezogen auf das Gesamtvolumen der Lösung, zugegeben. Der pH-Wert der Lösung wird kontrolliert und gegebenenfalls mit einigen Tropfen 1N NaOH auf pH 5,5 eingestellt. Die Lösung wird bei Raumtemperatur langsam gerührt, dabei fällt das Produkt in der gewünschten Form an. Zur Vervollständigung der Fällung wird über Nacht bei Kühlraumtemperatur gelagert. Nach Sedimentation des Produkts wird der klare Überstand abdekantiert. Das Sediment wird zentrifugiert. Das so erhaltene feuchte monosphärische Gly-A21-di-Arg-(B31-32)-Humaninsulin wird zur Entfernung anhaftender Puffer mit Wasser gewaschen und anschließend bei 0°C unter vermindertem Druck getrocknet. Ausbeute: 36 g.

### Beispiel 2

### Herstellung von monosphärischem Di-Arg-(B31-32)-Humaninsulin

Durch Fermentation von genetisch modifizierten Escherichia-coli-Zellen wird wie im Beispiel 1 ein Insulinderivat 1 mit folgender Aminosäuresequenz hergestellt. Insulin 2 (Seq Id No. 4):

Phe Val Asn Gln His Leu Cys Gly Ser His Leu Val Glu Ala Leu Tyr Leu Val
Cys Gly Glu Arg Gly Phe Phe Tyr Thr Pro Lys Thr Arg Arg Gly Ile Val Glu
Gln Cys Cys Thr Ser Ile Cys Ser Leu Tyr Gln Leu Glu Asn Tyr Cys Asn

Insulin 2 entspricht der Formel I, dabei ist
- X: Arg-Arg
- n: die Zahl 2,
- Y: Thr (B30),
- R¹: Phe (B1),
- R²: Asn (A21) und

A2 - A20 die Aminosäuresequenz der A-Kette von Humaninsulin (Aminosäurereste 2 bis 20) und B2 - B29 die Aminosäuresequenz der B-Kette von Humaninsulin (Aminosäurereste 2 bis 29).

1 g Di-Arg-(B31-32)-Humaninsulin werden in 100 ml Puffer B gelöst und die Lösung sterilfiltriert. Das Filtrat wird mit 100 ml von sterilem Puffer A und 350 ml sterilem Wasser verdünnt und 3 Stunden bei Raumtemperatur mit einem Blattrührer gerührt. Zur Vervollständigung der Sedimentation wird die Suspension 14 Stunden bei 4°C gelagert. Das so gewonnene monosphärische Insulinderivat wird abzentrifugiert und der Niederschlag zur Entfernung anhaftenden Puffers mit Wasser gewaschen und lyophilisiert. Ausbeute: 0,87 g.
- Puffer A:: 0,2 M (NH₄)₂SO₄ / 0,1 M Glycin / 0,025 M Essigsäure in 5 % wäßrigem n-Propanol / pH 5,5
- Puffer B:: 0,05 M (NH₄)₂SO₄ / 0,1 M Glycin / 0,025 M Essigsäure in 50 % wäßrigem n-Propanol / pH 5,5

### Beispiel 3

### Herstellung einer Zubereitung von Gly-A21-di-Arg-(B31-32)-Humaninsulin

14,8 mg von steril hergestelltem Gly-A21-di-Arg-(B31-32)-Humaninsulin (monosphärisch, gemäß Beispiel 1) werden unter sterilen Bedingungen in 10 ml eines Placebo-Puffers für Insulinzubereitungen (pH 7) suspendiert. Zur Stabilitätsüberprüfung wurde die Suspension 1 Woche lang bei 4°C auf einem Schütteltisch mit 100 Hz bewegt. Die mikroskopische Untersuchung der sphärischen Form zeigte keine Veränderungen. Die chromatographische Untersuchung des klaren Überstandes nach Zentrifugation ergab weniger als 1 % Insulin in der Lösung.

Um die blutzuckersenkende Wirkung des obigen Insulinderivats zu testen wurde zunächst ein Teil der Zubereitung durch Ansäuern auf pH 4,5 in eine klare Lösung verwandelt. Intravenöse Applikation (0,2 IE/kg) am Hund zeigte eine Blutzuckersenkung die vergleichbar ist mit der einer äquimolaren Dosis an Humaninsulin. Subcutane Applikation der Suspension zeigte zunächst nur eine geringfügige aber lang andauernde Blutzuckersenkung, die nach wiederholter Injektion ein Basalprofil ergab.

## Patentansprüche

1. Amorphe monosphärische Form von Insulinderivaten, dadurch erhältlich, daß man
A) das Insulinderivat in einem n-Propanol-Puffer-Gemisch löst, wobei der n-Propanol-Gehalt bezogen auf Wasser mehr als 15 % und der pH-Wert 4,5 bis 6,5 beträgt, und
B) die erhaltene Lösung mit Wasser verdünnt, so daß der n-Propanol-Gehalt bezogen auf Wasser kleiner als 15 % ist, wobei eine gemäß Debye-Scherrer-Röntgenanalyse amorphe monosphärische Form des Insulinderivats aus der Lösung ausfällt.

2. Amorphe monosphärische Form eines Insulinderivats gemäß Anspruch 1, dadurch gekennzeichnet, daß man ein Insulinderivat der Formel I 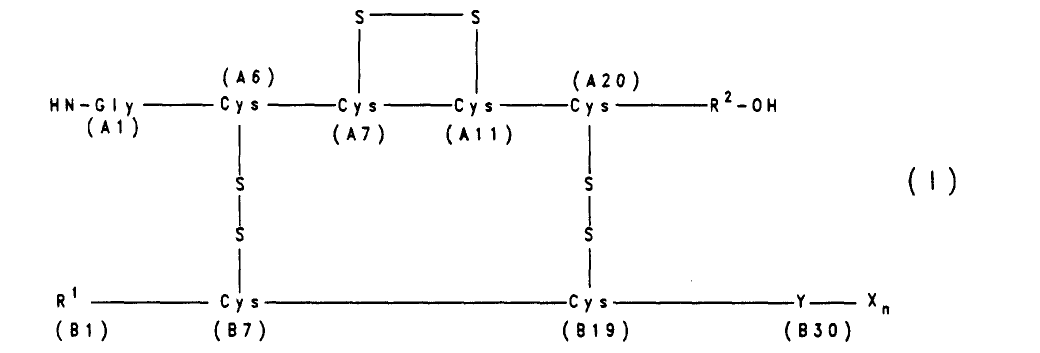 dabei ist
X ein genetisch kodierbarer Aminosäurerest,
n eine ganze Zahl von 1 bis 10,
Y ein genetisch kodierbarer Aminosäurerest,
R¹ ein Phenylalaninrest oder Wasserstoffatom,
R² ein genetisch kodierbarer Aminosäurerest und
die Reste A2 - A20 entsprechen der Aminosäuresequenz der A-Kette von Humaninsulin, tierischem Insulin oder einem Insulinderivat, und die Reste B2 - B 29 entsprechen der Aminosäuresequenz der B-Kette von Humaninsulin, tierischem Insulin oder einem Insulinderivat, einsetzt.

3. Amorphe monosphärische Form eines Insulinderivats gemäß Anspruch 1, dadurch gekennzeichnet, daß man ein Insulinderivat der Formel I, dabei ist
X ein Aminosäurerest von Arg oder Lys,
n eine ganze Zahl von 1 oder 2,
Y der Aminosäurerest Ala, Thr oder Ser,
R¹ der Aminosäurerest Phe,
R² der Aminosäurerest von Asn, Ser oder Gly,
und die Reste A2 - A20 und B2 - B29 entsprechen der Aminosäuresequenz der A- und B-Ketten von Humaninsulin, einsetzt.

4. Amorphe monosphärische Form eines Insulinderivats gemäß Anspruch 1, dadurch gekennzeichnet, daß man ein Insulinderivat der Formel I, dabei ist
X ein Aminosäurerest von Arg oder Lys,
n eine ganze Zahl von 1 oder 2,
Y der Aminosäurerest Thr,
R¹ der Aminosäurerest Phe,
R² der Aminosäurerest von Asn oder Gly,
und die Reste A2 - A20 und B2 - B29 entsprechen der Aminosäuresequenz der A- und B-Ketten von Humaninsulin, einsetzt.

5. Verfahren zur Herstellung einer amorphen, monosphärischen Form eines Insulinderivats gemäß der Definition in einem oder mehreren der Ansprüche 1 bis 4, dadurch gekennzeichnet, daß man
A) das Insulinderivat in einem n-Propanol-Puffer-Gemisch löst, wobei der n-Propanol-Gehalt bezogen auf Wasser mehr als 15 % und der pH-Wert 4,5 bis 6,5 beträgt, und
B) die erhaltene Lösung mit Wasser verdünnt, so daß der n-Propanol-Gehalt bezogen auf Wasser kleiner als 15 % ist, wobei eine Debye-Scherrer-Röntgenanalyse amorphe monosphärische Form des Insulinderivats aus der Lösung ausfällt.

6. Verfahren nach Anspruch 5, dadurch gekennzeichnet, daß man die Propanolkonzentration nach der Verdünnung 5 bis 12 % beträgt.

7. Verfahren nach Anspruch 5, dadurch gekennzeichnet, daß man einen Puffer, der Glycin, Ammoniumsulfat und Essigsäure enthält, einsetzt.

8. Pharmazeutische Mittel, enthaltend die amorphe monosphärische Form eines Insulinderivats gemäß einem oder mehreren der Ansprüche 1 bis 4 oder wie erhalten nach Ansprüchen 5 bis 7 und einen physiologisch verträglichen Träger.

9. Pharmazeutische Mittel gemäß Anspruch 8 zur Behandlung von Diabetes mellitus.

10. Verwendung der amorphen monosphärischen Form eines Insulinderivats gemäß einem oder mehreren der Ansprüche 1 bis 4 oder wie erhalten nach Ansprüchen 5 bis 7 sowie der pharmazeutischen Mittel gemäß Anspruch 8 oder 9 zur Behandlung von Diabetes mellitus.

## Claims

1. An amorphous monospheric form of insulin derivatives, obtainable when
A) the insulin derivative is dissolved in an n-propanol/buffer mixture in which the n-propanol content relative to water is more than 15% and the pH is from 4.5 to 6.5, and
B) the resulting solution is diluted with water so that the n-propanol content relative to water is less than 15%, whereupon an insulin derivative which is in an amorphous monospheric form as shown by Debye-Scherrer X-ray analysis precipitates out of the solution.

2. An amorphous monospheric form of an insulin derivative as claimed in claim 1, wherein an insulin derivative of the formula I 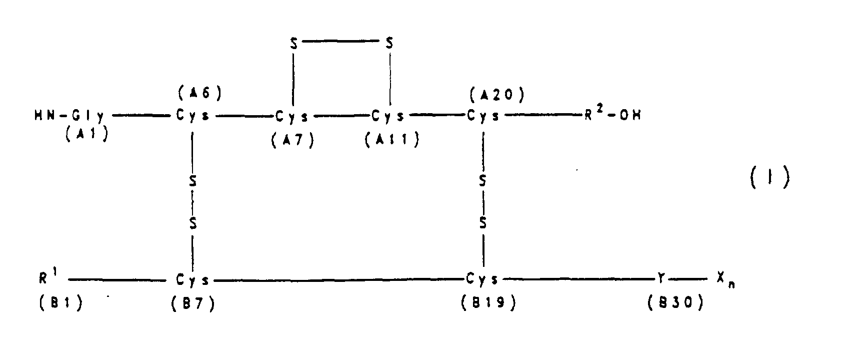 in which
X is a genetically encodable amino acid residue,
n i is an integer from 1 to 10,
Y ' is a genetically encodable amino acid residue,
R¹ is a phenylalanine residue or a hydrogen atom,
R² is a genetically encodable amino acid residue and
the residues A2 - A20 correspond to the amino acid sequence of the A chain of human insulin, animal insulin or an insulin derivative, and the residues B2 - B29 correspond to the amino acid sequence of the B chain of human insulin, animal insulin or an insulin derivative, is used.

3. An amorphous monospheric form of an insulin derivative as claimed in claim 1, wherein an insulin derivative of the formula I, in which
X is an Arg or Lys amino acid residue,
n is an integer 1 or 2,
Y is the Ala, Thr or Ser amino acid residue,
R¹ is the Phe amino acid residue,
R² is the Asn, Ser or Gly amino acid residue,
and the residues A2 - A20 and B2 - B29 correspond to the amino acid sequence of the A and B chains of human insulin, is used.

4. An amorphous monospheric form of an insulin derivative as claimed in claim 1, wherein an insulin derivative of the formula I, in which
X is an Arg or Lys amino acid residue,
n is an integer 1 or 2,
Y is the Thr amino acid residue,
R¹ is the Phe amino acid residue,
R² is the Asn or Gly amino acid residue,
and the residues A2 - A20 and B2 - B29 correspond to the amino acid sequence of the A and B chains of human insulin, is used.

5. A process for the production of an amorphous, monospheric form of an insulin derivative as defined in one or more of claims 1 to 4, which comprises
A) the insulin derivative being dissolved in an n-propanol/buffer mixture in which the n-propanol content relative to water is more than 15% and the pH is from 4.5 to 6.5,
and
B) the resulting solution being diluted with water so that the n-propanol content relative to water is less than 15%, whereupon an insulin derivative which is in an amorphous monospheric form as shown by Debye-Scherrer X-ray analysis precipitates out of the solution.

6. The process as claimed in claim 5, wherein the propanol concentration after the dilution is 5 to 12%.

7. The process as claimed in claim 5, wherein a buffer containing glycine, ammonium sulfate and acetic acid is used.

8. A pharmaceutical composition, containing the amorphous monospheric form of an insulin derivative as claimed in one or more of claims 1 to 4 or as obtained as claimed in claims 5 to 7 and a physiologically tolerated vehicle.

9. A pharmaceutical composition as claimed in claim 8 for the treatment of diabetes mellitus.

10. The use of the amorphous monospheric form of an insulin derivative as claimed in one or more of claims 1 to 4 or as obtained as claimed in claims 5 to 7, and of the pharmaceutical compositions as claimed in claim 8 or 9 for the treatment of diabetes mellitus.

## Revendications

1. Forme monosphérique amorphe de dérivés d'insuline, que l'on peut obtenir
A) en dissolvant le dérivé d'insuline dans un mélange de tampon et de n-propanol, la teneur en n-propanol, par rapport à l'eau, étant supérieure à 15 %, et le pH étant de 4,5 à 6,5, et
B) en diluant avec de l'eau la solution obtenue, de sorte que la teneur en n-propanol, par rapport à l'eau, est inférieure à 15 %, ce par quoi une forme monosphérique amorphe du dérivé d'insuline, selon analyse aux rayons X par la méthode de Debye-Scherrer, précipite dans la solution.

2. Forme monosphérique amorphe d'un dérivé d'insuline selon la revendication 1, caractérisée en ce que l'on utilise un dérivé d'insuline de formule I 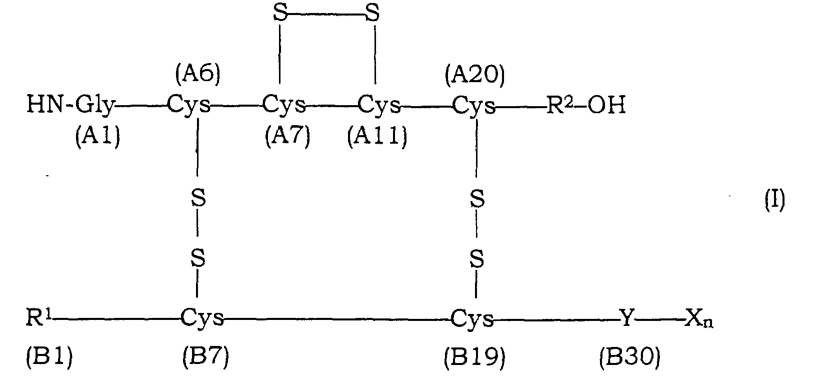 dans laquelle
X est un résidu aminoacide génétiquement codable,
n est un nombre entier allant de 1 à 10,
Y est un résidu aminoacide génétiquement codable,
R¹ est un résidu phénylalanine ou un atome d'hydrogène,
R² est un résidu aminoacide génétiquement codable, et
les résidus A2 - A20 correspondent à la séquence d'aminoacides de la chaîne A de l'insuline humaine, d'une insuline animale ou d'un dérivé d'insuline, et les résidus B2 - B29 correspondent à la séquence d'aminoacides de la chaîne B de l'insuline humaine, d'une insuline animale ou d'un dérivé d'insuline.

3. Forme monosphérique amorphe d'un dérivé d'insuline selon la revendication 1, caractérisée en ce que l'on utilise un dérivé d'insuline de formule I, dans lequel
X est un résidu aminoacide de Arg ou Lys,
n est un nombre entier égal à 1 ou 2,
Y est un résidu aminoacide de Ala, Thr ou Ser,
R¹ est le résidu aminoacide Phe,
R² est un résidu aminoacide de Asn, Ser ou Gly, et
les résidus A2 - A20 et B2 - B29 correspondent à la séquence d'aminoacides des chaînes A et B de l'insuline humaine.

4. Forme monosphérique amorphe d'un dérivé d'insuline selon la revendication 1, caractérisée en ce que l'on utilise un dérivé d'insuline de formule I, dans lequel
X est un résidu aminoacide de Arg ou Lys,
n est un nombre entier égal à 1 ou 2,
Y est le résidu aminoacide Thr,
R¹ est le résidu aminoacide Phe,
R² est le résidu aminoacide de Asn ou Gly, et
les résidus A2 - A20 et B2 - B29 correspondent à la séquence d'aminoacides des chaînes A et B de l'insuline humaine.

5. Procédé pour la préparation d'une forme monosphérique amorphe d'un dérivé d'insuline selon la définition dans une ou plusieurs des revendications 1 à 4, caractérisé en ce que
A) on dissout le dérivé d'insuline dans un mélange de tampon et de n-propanol, la teneur en n-propanol, par rapport à l'eau, étant supérieure à 15 %, et le pH étant de 4,5 à 6,5, et
B) on dilue avec de l'eau la solution obtenue, de sorte que la teneur en n-propanol, par rapport à l'eau, est inférieure à 15 %, ce par quoi une forme monosphérique amorphe du dérivé d'insuline, selon analyse aux rayons X par la méthode de Debye-Scherrer, précipite dans la solution.

6. Procédé selon la revendication 5, caractérisé en ce que la concentration de propanol après la dilution est de 5 à 12 %.

7. Procédé selon la revendication 5, caractérisé en ce que l'on utilise un tampon qui contient de la glycine, du sulfate d'ammonium et de l'acide acétique.

8. Produits pharmaceutiques contenant la forme monosphérique amorphe d'un dérivé d'insuline selon une ou plusieurs des revendications 1 à 4 ou telle qu'obtenue selon les revendications 5 à 7, et un véhicule physiologiquement acceptable.

9. Produits pharmaceutiques selon la revendication 8, destinés au traitement du diabète sucré.

10. Utilisation de la forme monosphérique amorphe d'un dérivé d'insuline selon une ou plusieurs des revendications 1 à 4 ou telle qu'obtenue selon les revendications 5 à 7, ainsi que des produits pharmaceutiques selon la revendication 8 ou 9, pour le traitement du diabète sucré.
